# EUROPÄISCHE PATENTSCHRIFT

(11) **EP 2 241 272 B1**
(45) Veröffentlichungstag und Bekanntmachung des Hinweises auf die Patenterteilung: **16.05.2012**
(21) Anmeldenummer: 10160031.0
(22) Anmeldetag: 15.04.2010
(51) Int. Cl.: A61B 17/28, A61B 17/29, A61B 17/00

(54) **Medizinisches Instrument mit einer drehbaren Rastsperre**
Medicinal instrument with a rotatable break block
Instrument médical doté d'une fermeture à cran d'arrêt rotative

(30) Priorität: 17.04.2009 DE 102009018638
(43) Veröffentlichungstag der Anmeldung: 20.10.2010
(73) Patentinhaber: Karl Storz GmbH & Co. KG, 78532 Tuttlingen (DE)
(72) Erfinder: Bacher, Uwe, 78532, Tuttlingen (DE); Summerer, Sabine, 85560 Ebersberg (DE)
(74) Vertreter: Weller, Wolfgang

(56) Entgegenhaltungen:
- DE-A1- 10 125 149
- US-A1- 2007 299 469
- US-A1- 2008 154 299

## Beschreibung

Die Erfindung betrifft ein medizinisches Instrument mit einem Schaft der proximal mit einer Handhabe verbunden ist, mit einem längs des Schafts geführten Einsatz, der proximalseitig mit einem beweglichen Griffteil der Handhabe verbunden ist und distal ein Werkzeug aufweist, das durch Bewegen des Griffteils betätigbar ist, und mit einer Raste durch die das bewegliche Griffteil in einer Stellung arretierbar ist, wobei eine Rastsperre vorgesehen ist, durch die die Arretierfunktion dauerhaft aufhebbar ist, wobei die Rastsperre ein drehbares Abstellorgan aufweist, das am beweglichen Griffteil angeordnet ist, wobei das Abstellorgan in einer ersten Drehstellung die Arretierfunktion der Raste nicht beeinflusst, in einer zweiten Drehstellung die Raste dauerhaft aus dem arretierenden Eingriff weghält.

Ein derartiges medizinisches Instrument ist aus der US 2008/154299 A1 bekannt.

Ein anderes medizinisches Instrument in Form einer Präparier- oder Fasszange ist aus dem Katalog der Anmelderin Laparoskopie, 5. Ausgabe 1/2005 Kapitel 4 "Präparier- und Fasszangen", Seite 77 bekannt.

Mittels der Raste kann das bewegliche Griffteil, das das Werkzeug betätigt, fixiert werden. Die Raste besteht dabei meist aus einem stabförmigen Bauteil, das an einem unbeweglichen Griffteil angebracht ist. Die Raste weist auf der Seite, die dem beweglichen Griffteil zugewandt ist, Zähne auf, die mit einem Rastelement am beweglichen Griffteil zusammenwirken.

Weitere Instrumente mit solchen Rasten sind beispielsweise aus der DE 102 32 086 A1, und aus der WO 2006/071120 A1 bekannt.

Derartige medizinische Instrumente finden insbesondere in der minimalinvasiven Chirurgie Anwendung und werden im Hinblick auf Einsatz und Verstellmöglichkeiten immer weiter entwickelt.

Eine Weiterentwicklung besteht beispielsweise darin, den starren Schaft zumindest in seinem distalen Endbereich abwinkelbar auszubilden.

Derartige Vorrichtungen sind beispielsweise aus der US 5,618,294, und der US 6,077,287 bekannt. Die Abwinkelbarkeit wird dadurch erreicht, dass der distale Endbereich flexibel aufgebaut wird, beispielsweise in Form von wirbelartigen miteinander verbundenen Elementen. Die Steuerung, also die Abwinkelung und die wieder geradlinige Ausrichtung wird über diametral im Schaft angeordnete Seilzüge bewerkstelligt.

Dazu sind zahlreiche Stellorgane im Bereich der Handhabe bzw. der Griffteile vorhanden. D.h. Stellorgane um den Schaft auszulenken, Stellorgane um das Griffteil zu bewegen, Stellorgane um die Rastfunktion zu öffnen und zu schließen und dergleichen. Es besteht ein Bestreben, diese Stellorgane möglichst im Bereich des Handgriffes anzuordnen, so dass eine Bedienungsperson, die das Instrument an der Handhabe ergriffen hat, mit den Fingern die verschiedenen Stellorgane bedienen kann.

Hier ist aber darauf zu achten, dass diese Stellorgane möglichst ergonomisch angeordnet sind und auch eindeutig sind, damit nicht während eines operativen Eingriffes versehentlich ein falsches Stellorgan bedient wird.

Bei kombinierten Präparier-/Fasszangen ist es wünschenswert, dass solange der Präpariervorgang abläuft, die Maulteile des Werkzeuges frei bewegt werden können, dieser soll also nicht durch eine versehentlich ausgelöste andere Funktion gestört werden.

Nach dem Präparieren, beispielsweise einem Abtrennen eines Gewebestückes, ist es dann wünschenswert, dieses abgetrennte Gewebestück zwischen den Maulteilen zu fassen, wobei ein gewisser Anpress- und Haltedruck ausgeübt werden muss und in diesem Zustand soll das Gewebe gehalten werden. Dieses Halten soll sicherstellen, dass bei weiteren Manipulationen das erfasste Gewebestück nicht verlorengeht, sondern dass es bei einem minimalinvasiven Eingriff, beispielsweise durch eine Trokarhülse, die im Körper steckt, vom Körper abgezogen werden kann. Wurde beispielsweise der Präparier- und anschließende Fassvorgang in einem abgewinkelten Zustand des Schaftes durchgeführt, muss dieser zunächst geradlinig ausgerichtet werden, damit er über eine Trokarhülse abgezogen werden kann.

Es ist daher Aufgabe der vorliegenden Erfindung, ein medizinisches Instrument, insbesondere solche mit einem abwinkelbaren Schaft, dahingehend weiterzuentwickeln, dass eine funktionssichere und ergonomische Handhabung möglich ist, insbesondere, was die Arretierung und die Lösung der Arretierung des beweglichen Griffteils betrifft.

Erfindungsgemäß wird die Aufgabe dadurch gelöst, dass die Drehebene des Abstellorganes quer zur Längsachse der Bewegungsrichtung des Griffteiles verläuft.

Diese Maßnahmen haben nun im Hinblick auf Funktionssicherheit und Ergonomie mehrere Vorteile. Die Anordnung des drehbaren Abstellorgans am bewegbaren Griffteil hat den Vorteil, dass sich in allen Verschwenk-, Verschiebe- oder sonstigen Stellungen des beweglichen Griffteils das Abstellorgan immer in einer bestimmten Relativposition dazu befindet Die Handhabungsperson kann das bewegliche Griffteil, das beispielsweise die Maulteile öffnet und schließt, in gewohnter Manier hin- und herbewegen, sei es Verschwenken oder linear hin- und herbewegen, ohne dass dabei das drehbare Abstellorgan benötigt wird. Soll die Arretierfunktion auf Dauer aufgehoben werden, kann man das drehbare Abstellorgan an dem bewegbaren Griffteil ertasten und entsprechend verdrehen. Dadurch, dass die Drehebene quer zur Längsachse der Bewegungsrichtung des Griffteiles verläuft, kann beim Bewegen des Griffteiles das drehbare Abstellorgan nicht versehentlich bewegt werden, denn dazu muss mit einem Finger der Hand eine völlig andere Bewegung durchgeführt werden. Üblicherweise haben Griffteile einen Fingerring, in den der Finger eingesteckt wird, um dieses zu bewegen. Die Handhabungsperson muss nun entweder ganz definitiv einen anderen Finger heranziehen, um das drehbare Abstellorgan zu bewegen oder zunächst den bereits im Fingerring steckenden Finger von diesem abziehen und damit das drehbare Abstellorgan bewegen. Hiermit ist vorrangig der Betriebssicherheit Genüge getan.

Die Drehbewegung als solches ist eine Bewegung, die ruckfrei und ergonomisch durchgeführt werden kann. Das Vorsehen von zwei verschiedenen Stellungen, in denen zwei verschiedene Funktionen möglich sind, nämlich einmal die Arretierfunktion der Raste nicht zu beeinflussen bzw. in der zweiten Stellung die Raste dauerhaft aus dem arretierenden Eingriff wegzuhalten, ist einfach, somit ergonomisch. Diese sind sicher von der Handhabungsposition zu ertasten bzw. zu er- und begreifen. Wirkt die Raste, also sind die Griffteile relativ zueinander verrastet, weiß man, dass sich das drehbare Abstellorgan in der ersten Stellung befindet, denn in dieser Stellung hat die Rastsperre keinen Einfluss auf die Arretierfunktion der Raste. Man muss dann das drehbare Abstellorgan nur soweit bewegen, bis man die zweite Position erreicht hat, was einfach beispielsweise durch einen Anschlag angezeigt werden kann. Es ist dann sichergestellt, dass nunmehr die Raste dauerhaft aus dem arretierenden Eingriff weggehalten ist. Man spürt das dadurch, dass man das bewegliche Griffteil ohne Funktion der Raste bewegen kann. Das kann man auch ohne große Aufmerksamkeit, spricht ohne Blickkontakt durchführen, denn die ergonomische Anordnung des drehbaren Abstellorgans am beweglichen Griffteil erlaubt das drehbare Abstellorgan ohne Blickkontakt entsprechend zu verdrehen.

Somit sind beiden Anforderungen, also sowohl der Betriebssicherheit als auch der Ergonomie Genüge getan.

In einer weiteren Ausgestaltung der Erfindung ist das drehbare Abstellorgan als Drehring ausgebildet, der distal vor einem Fingerring des beweglichen Griffteiles angeordnet ist.

Diese Ausgestaltung ist mechanisch einfach, ein Drehring kann ruckfrei und sanft bewegt werden. Die Anordnung distal vor dem Fingerring erlaubt es der Handhabungsperson diesen Drehring entweder mit demjenigen Finger zu verdrehen, der in dem Fingerring steckt oder mit dem nächst daneben liegenden Finger. Hat man beispielsweise den Zeigefinger in den Fingerring des beweglichen Griffteiles eingeführt, kann man problemlos mit dem Mittelfinger den distal davor angeordneten Drehring bewegen.

In einer weiteren Ausgestaltung der Erfindung steht vom Drehring radial ein Stab vor, über den der Drehring drehbar ist.

Diese Maßnahme hat den Vorteil, dass ein solcher abstehender Stab sehr einfach ohne Blickkontakt ertastet werden kann und mit einem Finger bewegt werden kann. Dies ist als eine besonders ergonomisch günstige Ausgestaltung anzusehen.

In einer weiteren Ausgestaltung der Erfindung erstreckt sich der Stab in der ersten Drehstellung in einer Ebene des Fingerrings des beweglichen Griffteils.

Diese Maßnahme hat den erheblichen Vorteil, dass sich der Stab quasi hinter bzw. vor der Fingerringebene erstreckt und versteckt, somit Manipulationen wie das Ein- und Ausschieben des Fingers in den Fingerring nicht hindert.

In einer weiteren Ausgestaltung der Erfindung erstreckt sich der Stab in der zweiten Stellung seitlich vom beweglichen Griffteil weg.

In ergonomischer Hinsicht ist das besonders einfach und günstig zu erzielen. Wenn beispielsweise in dem beweglichen Griffteil der Zeigefinger steckt, kann dieses seitliche Verdrehen des Ringes sehr einfach mit dem Mittelfinger durchgeführt werden, in dem dieser seitlich an den Stab angelegt wird und diesen zur Seite verschwenkt.

In dieser seitlich verschwenkten Position kann der Stab auch einfach ertastet und ergriffen werden, um dann bei der umgekehrten Bewegung, nämlich wieder sicher und ergonomisch an das Griffteil bewegt zu werden.

In einer weiteren Ausgestaltung der Erfindung beträgt der Verschwenkbereich ca. 90°.

Dieser Verschwenkbereich gibt der Handhabungsperson ein gutes Feedback über die beiden Stellungen, d.h. er muss den Hebel oder den Drehring nur zwischen diesen beiden, etwa um 90° verschwenkten Positionen hin- und herbewegen. Das ist wiederum sehr einfach mit einem Finger einer Hand zu bewerkstelligen, die ohnehin das Instrument an der Handhabe hält.

In einer weiteren Ausgestaltung der Erfindung ist im bewegbaren Griffteil ein Mechanismus angeordnet, der in der ersten Drehstellung des Abstellorgans eine Aufhebung der Arretierfunktion der Raste ermöglicht.

Diese Maßnahme hat den erheblichen Vorteil, dass über den zusätzlichen Mechanismus eine kurzfristige Aufhebung der Arretierfunktion möglich ist, wenn beispielsweise die Handhabungsperson die Stellung der Maulteile etwas korrigieren möchte. In diesem Fall muss nicht das drehbare Abstellorgan verdreht werden, sondern man kann kurzfristig, beispielsweise für nur ein oder zwei Sekunden den Mechanismus betätigen, der dann für diesen kurzen Zeitraum die Arretierfunktion der Raste löst.

Wird dieser Mechanismus wieder freigegeben, kann die Raste sofort wieder in die Rastposition einfahren und kann wieder die Arretierfunktion übernehmen. Dies ist ja deswegen möglich, da das drehbare Abstellorgan in der ersten Drehstellung die Arretierfunktion der Raste nicht beeinflusst.

In einer weiteren Ausgestaltung der Erfindung weist der Mechanismus einen Kipphebel auf, der mit der Raste in Wirkverbindung steht.

Diese Maßnahme hat nun in ergonomischer Hinsicht den Vorteil, dass ein Kipphebel eine andere Mechanik darstellt als ein drehbares Abstellorgan, so dass hier wieder sichergestellt ist, dass durch Ertasten und Bewegen leicht erkennbar ist, welches Stellorgan man zur Aufhebung der Arretierfunktion bedient, nämlich den Kipphebel zum kurzfristigen Aufheben oder das drehbare Abstellorgan zum dauerhaften Aufheben. Dies trägt insbesondere zur Funktionssicherheit bei.

In einer weiteren Ausgestaltung der Erfindung weist der Kipphebel eine sich im Fingerring des beweglichen Griffteils angeordnete Taste auf.

In ergonomischer Hinsicht hat es den Vorteil, dass mit dem Finger, der in dem Fingerring steckt, die Taste betätigt werden kann, um kurzfristig die Rastwirkung aufzuheben. Lässt man die Taste los, rastet die Raste sofort wieder ein. Will die Handhabungsperson die Rastfunktion auf Dauer aufheben, wird die Rastsperre in Form des drehbaren Abstellorganes bewegt. Dieses liegt benachbart zu dem Fingerring des beweglichen Griffteiles, so dass dadurch keine Verwechslungen stattfinden können, was die Betriebssicherheit erhöht. Dies ist auch sehr ergonomisch, da dies für die Handhabungsperson zwei völlig unterschiedliche Bauelemente sind, nämlich zum einen eine Taste, zum andern ein drehbarer Ring, was durch Tastfunktion bzw. haptische Empfindungen einfach festzustellen ist. Somit trägt diese Ausgestaltung auch besonders der Ergonomie bei.

Wie bereits erwähnt, ist von besonderem Vorteil, dass durch ein Drücken der Taste die Raste aus der arretierenden Position abhebt und die Raste bei Freigabe der Taste wieder in die arretierende Position bewegt wird.

In einer weiteren Ausgestaltung der Erfindung ist die Raste als zweiarmiger Hebel ausgebildet, wobei ein erster Hebelarm Rastzähne trägt, die mit einem Rastelement an der Handhabe in arretierenden Eingriff treten.

Diese Maßnahme hat den erheblichen Vorteil, dass durch ein konstruktiv einfach ausgebildetes Element, nämlich den zweiarmigen Hebel die Raste ausgebildet ist und ein Verrasten bzw. eine Lösung der Verrastung durch eine einfachen Verschwenkung des Hebels bewerkstelligt werden kann.

In einer weiteren Ausgestaltung der Erfindung ist das bewegbare Griffteil längs einer linearen Führung längs der Handhabe bewegbar, und die Rastzähne des zweiarmigen Hebels, der am beweglichen Griffteil angeordnet ist, sind dem Rastelement zugewandt.

Diese Ausgestaltung erlaubt eine kompakte und betriebssichere Bauweise.

In einer weiteren Ausgestaltung der Erfindung ist die Raste durch eine Feder derart vorgespannt, dass diese in den arretierenden Eingriff gedrückt wird.

Diese Maßnahme hat den Vorteil, dass eine Zwangssteuerung der Raste in die verriegelnde Stellung besteht.

In einer weiteren Ausgestaltung der Erfindung weist das drehbare Abstellorgan eine umfängliche radial ansteigende Nockenfläche auf, die beim Drehen aus der ersten in die zweite Drehstellung den ersten Hebelarm mit den Rastzähnen vom Rastzahn abhebt.

Diese Maßnahme hat den Vorteil, dass durch eine solche Nockenfläche eine ruckfreie Abhebebewegung des ersten Hebelarms mit den Rastzähnen von dem Rastzahn erfolgt.

Dies ist eine harmonische und keine ruckartige Bewegung und somit sehr ergonomisch.

In einer weiteren Ausgestaltung der Erfindung tritt die Nockenfläche mit dem zweiten Hebelarm des zweiarmigen Hebels in Eingriff.

Diese Maßnahme hat den Vorteil, dass der Hebelarm des zweiarmigen Hebels, der mit den Rastzähnen versehen ist, nicht mit der Nockenfläche in Eingriff steht, sondern der gegenüberliegende zweite Hebelarm des zweiarmigen Hebels. Somit steht der gesamte erste Hebelarm für die Rastfunktion zur Verfügung.

In einer weiteren Ausgestaltung der Erfindung steht der Kipphebel mit dem ersten Hebelarm des zweiarmigen Hebels in Eingriff.

Diese Maßnahme hat konstruktiv und bezüglich der Betriebssicherheit den erheblichen Vorteil, dass durch die mechanische Verbindung des Kipphebels mit dem ersten Hebelarm, der die Rastzähne trägt, eine unmittelbare mechanische Verbindung besteht, um die Raste kurzfristig aus dem arretierenden Eingriff abzuheben. Da dies in der ersten Stellung des drehbaren Abstellorgans abläuft, in der die Arretierfunktion der Raste nicht beeinflusst wird, kann also der zweite Hebel von der Nockenfläche frei abheben. Erst wenn das drehbare Abstellorgan verdreht wird, wird die Raste zwangsweise auf Dauer aus dem arretierenden Eingriff gebracht, d.h. das drehbare Abstellorgan übersteuert in diesem Sinne dann die Steuerungsfunktion des Kipphebels für das kurzfristige Lösen der Arretierung.

Somit ist das Zusammenwirken dieser beiden Mechanismen sehr funktionssicher und ist auch entsprechend ergonomisch durchzuführen, denn durch diese Übersteuerung braucht man nicht dafür Sorge zu tragen, dass auch noch der Kipphebel zusätzlich bewegt werden muss. Dieser wird durch den zweiarmigen Hebel dann automatisch mitbewegt, wenn der zweite Hebelarm durch die Nockenfläche bewegt wird.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird anhand eines ausgewählten Ausführungsbeispiels in Zusammenhang mit den beiliegenden Zeichnungen näher beschrieben und erläutert. Es zeigen:
- Fig. 1: eine Seitenansicht eines medizinischen Instrumentes, das mit einer erfindungsgemäßen Rastsperre versehen ist,
- Fig. 2: einen Schnitt durch das bewegliche Griffteil des Instrumentes von Fig. 1, das den Rastsperrenmechanismus aufweist, und zwar in der ersten Stellung der Rastsperre,
- Fig. 3: eine stark vergrößerte Darstellung des in Fig. 2 mit einem Kreis umgrenzten Bereiches,
- Fig. 4: eine stirnseitige Ansicht des Griffteils von Fig. 2 von distal nach proximal,
- Fig. 5: eine der Fig. 2 vergleichbare Darstellung, wobei sich der Rastsper- renmechanismus in der zweiten Stellung befindet,
- Fig. 6: eine stark vergrößerte Darstellung des in Fig. 5 mit einem Kreis umgrenzten Bereiches,
- Fig. 7: eine der Fig. 4 vergleichbare stirnseitige Ansicht des Griffteiles mit etwa um 90° verschwenktem Abstellorgan,
- Fig. 8: eine Draufsicht auf das bewegliche Griffteil in der in Fig. 2 darge- stellten ersten Stellung der Rastsperre, und
- Fig. 9: einen ausschnittsweisen Teilschnitt des Instrumentes von Fig. 1 im Bereich des beweglichen Griffteiles, wobei hier die Rastsperre in ih- rer zweiten Drehstellung befindlich dargestellt ist.

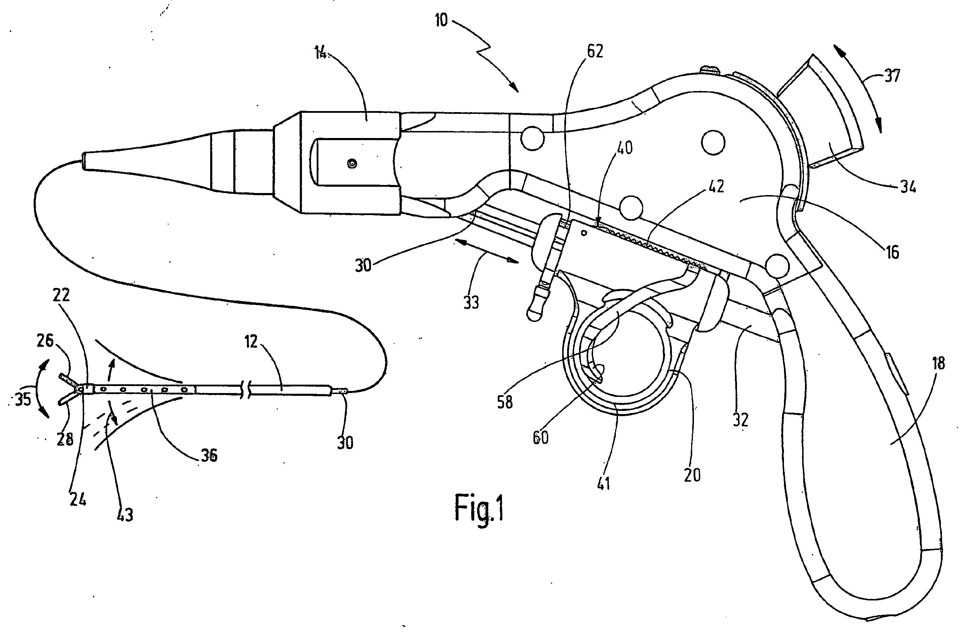

Ein in Fig. 1 dargestelltes medizinisches Instrument ist in seiner Gesamtheit mit der Bezugsziffer 10 bezeichnet.

Das Instrument 10 weist einen langerstreckten Schaft 12 auf, der proximalseitig mit einer Handhabe 14 verbunden ist. Die Handhabe weist ein Gehäuse 16 auf, von dem ein feststehendes Griffteil 18 vorsteht.

Am Gehäuse 16 ist ein bewegliches Griffteil 20 angeordnet, das längs einer Führung 32 hin- und herbewegt werden kann, wie das durch eine Doppelpfeil 33 angezeigt ist.

Im Schaft 12 ist ein Werkzeugeinsatz 22 aufgenommen, der distalseitig ein Werkzeug 24 trägt. Das Werkzeug 24 weist zwei spreizbare Maulteile 26 und 28 auf, die als kombinierte Präparier-/Fasszange arbeiten. Der Werkzeugeinsatz 22 weist ein stab- bzw. drahtförmiges Betätigungsorgan 30 auf, das sich von dem Werkzeug 24 im Inneren des Schaftes 12 bis in das Gehäuse 16 hinein, dort durch die als Rohr ausgebildete Führung 32 hindurch bis zum beweglichen Griffteil 20 erstreckt. Das proximale Ende des Betätigungsorganes 30 ist mit dem beweglichen Griffteil 20 verbunden.

Ferner ist zu entnehmen, dass an der Oberseite des Gehäuses 16 noch ein Stellorgan 34 vorsteht, das längs einer hier nicht bezeichneten Kreisbahn hin- und herverschoben werden kann, wie das durch einen Doppelpfeil 37 dargestellt ist. Das Stellorgan 34 ist mit zwei Steuerseilen verbunden, die ebenfalls durch das Gehäuse 16 und durch den Schaft 12 hindurch bis zu einem ablenkbaren Abschnitt 36 am distalen Ende des Schaftes 12 geführt sind. Diese Steuerseile sind hier nicht näher aufgezeigt. Wird das Stellorgan 34 in eine Richtung des Doppelpfeiles 37 bewegt, wird eines der beiden Steuerseile auf eine Trommel oder einem Trommelabschnitt aufgewickelt und das andere gleichzeitig abgewickelt. Dadurch erfolgt eine Auslenkung des ablenkbaren Abschnitts 36 beispielsweise in Richtung des Pfeiles 43. Wird das Stellorgan 34 in die entgegengesetzte Richtung bewegt, wird der ausgelenkte Abschnitt zunächst wieder gerade ausgerichtet, bzw. bei einer weiteren Bewegung dann in der entgegengesetzten Richtung ausgelenkt.

Im Betrieb ergreift eine Person das Instrument 10 mit einer Hand am feststehenden Griffteil 18 und steckt einen Finger, beispielsweise den Mittelfinger in einen Fingerring 41 des beweglichen Griffteiles hinein. Mit dem Daumen kann das Stellorgan 34 bewegt werden, um den ablenkbaren Abschnitt 36 am distalen Endbereich des Schaftes entsprechend, wie zuvor erwähnt, auszulenken. Durch Hin- und Herbewegen des beweglichen Griffteiles 20 längs der Führung 32 wird das Betätigungsorgan 30 linear verschoben und öffnet und schließt dabei, je nach Richtung, die Maulteile 26 und 28, wie das durch den Doppelpfeil 35 angedeutet ist.

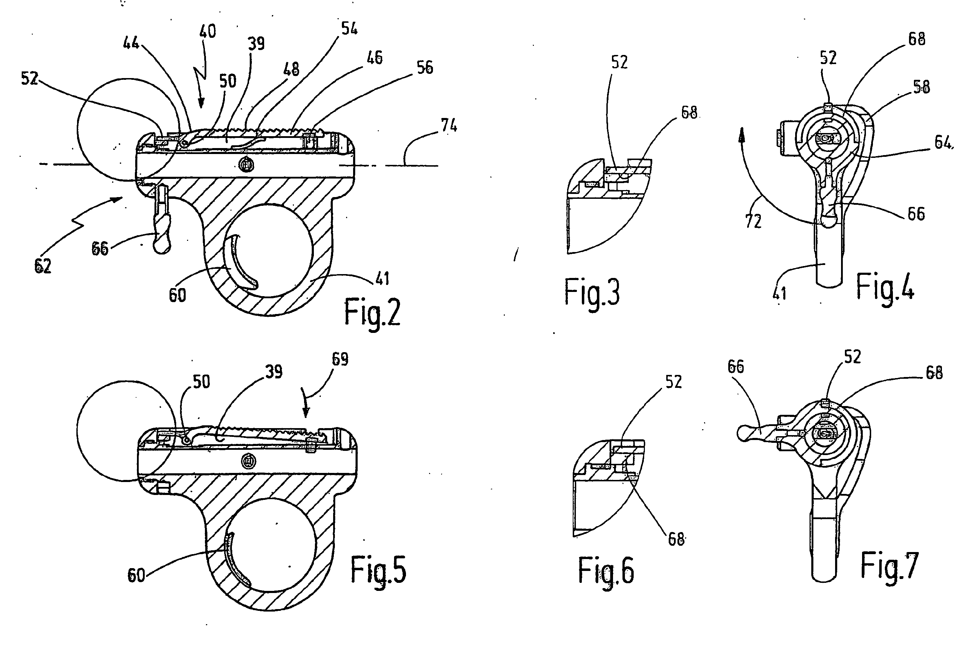

Aus Fig. 1 und insbesondere aus der Schnittdarstellung von Fig. 2 ist zu erkennen, dass im Körper des beweglichen Griffteiles 20 eine Raste 40 angeordnet ist, die mit einem vom Gehäuse 16 in Richtung des beweglichen Griffteiles 20 bzw. deren Raste 40 vorstehendem Rastelement 42 zusammenwirkt.

Das Rastelement 42 ist schlicht und einfach als ein ortsfester einziger vorspringender Rastzahn ausgebildet.

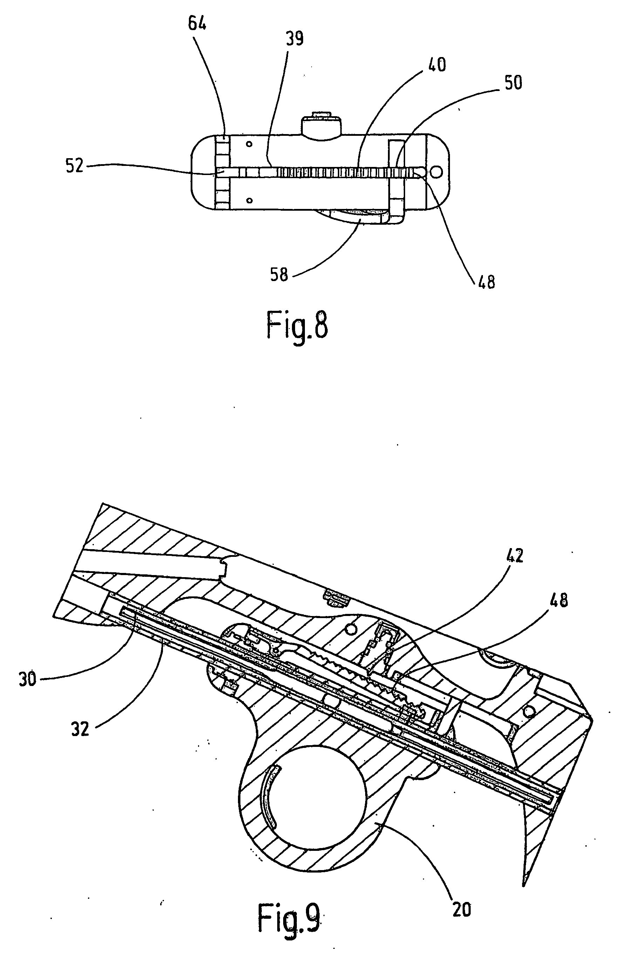

Wie aus der Schnittdarstellung von Fig. 2 bzw. von der Draufsicht von Fig. 8 zu erkennen, ist an der dem Gehäuse 16 zugewandten Ende des beweglichen Griffteiles 20 eine Aussparung 39 vorhanden, in der die Raste 40 aufgenommen ist.

Die Raste 40 ist als ein zweiarmiger Hebel 44 ausgebildet, dessen erster Hebelarm 46 auf der Seite, die dem Rastelement 42 zugewandt ist, eine Reihe an Rastzähnen 48 trägt. Der zweiarmige Hebel 44 ist um einen Schwenkzapfen 50 verschwenkbar. Diametral gegenüberliegend zum ersten Hebelarm 46 erstreckt sich ein allerdings wesentlich kürzerer zweiter Hebelarm 52 von dem Schwenkzapfen 50 weg.

Wie insbesondere aus Fig. 2 und Fig. 8 zu erkennen, erstreckt sich der zweiarmige Hebel in Richtung der Längsachse 74 der Führung 32, also in Richtung der Bewegung des beweglichen Griffteiles 20.

Das äußere Ende des ersten Hebelarmes 46 ist mit einem Kipphebel 56 fest verbunden, der einen sich seitlich des Fingerrings 41 erstreckenden Arm 58 aufweist, wie das insbesondere aus Fig. 1 und Fig. 4 ersichtlich ist. Der Arm 58 reicht durch den Fingerring 41 hindurch und zeigt endseitig eine Taste 60, die in den inneren Raum des Fingerrings 41 hineinragt.

Aus den Fig. 2 und 4 ist ersichtlich, dass distal vor dem Fingerring 41 das erfindungsgemäße Abstellorgan 62 angeordnet ist.

Das Abstellorgan 62 weist einen Ring 64 auf, von dem sich radial ein Stab 66 wegerstreckt.

Der Ring 64 ist in einer hier nicht näher bezeichneten Ringnut am beweglichen Griffteil eingelegt und umrundet den inneren Hohlraum der Führung 32.

Eine Außenseite des Ringes 64 ist als eine Nockenfläche 68 ausgebildet. Insbesondere aus dem vergrößerten Ausschnitt von Fig. 3 ist ersichtlich, dass die Unterseite des zweiten Hebelarmes 52 auf der Nockenfläche 68 aufliegt. Dieses Aufliegen erfolgt definiert, u.a. auch deswegen, weil sich zwischen der Unterseite des ersten Hebelarms 46 und dem Boden der Aussparung 39 eine Feder 54 befindet, die so vorgespannt ist, dass sie den ersten Hebelarm 46 in Richtung des Gehäuses 16 bzw. dessen Rastelement 42 drückt. D.h. die Feder 54 spannt die Raste 40 in Richtung arretierende Stellung vor. Der Hebel 44 selbst kann als Druckfeder ausgebildet sein, oder es kann eine Feder um dessen Schwenkzapfen 50 gelegt sein.

In der in Fig. 1 und 2 dargestellten Position befindet sich das Abstellorgan 62 in seiner ersten Stellung, in der die Arretierfunktion der Raste 40 nicht beeinflusst wird.

D.h., die Raste wird wie zuvor erwähnt durch die Feder 54 in Richtung arretierendem Eingriff mit dem Rastelement 52 gedrückt, wie das beispielsweise in Fig. 1 dargestellt ist.

Durch Drücken der Taste 60 kann der Kipphebel 56 bewegt werden, dieser zieht bei dieser Kippbewegung den ersten Hebelarm 46 der Raste 40 etwas in die Aussparung 39 hinein, somit wird dieser aus dem arretierenden Eingriff mit dem Rastelement 42 herausbewegt.

Bei dieser Bewegung kann der zweite Hebelarm 52 von der Nockenfläche, beispielsweise wie das aus Fig. 3 ersichtlich ist, problemlos abheben. Wird die Taste 60 wieder freigegeben, wird der erste Hebelarm 46 wieder durch die Feder 54 in den arretierenden Eingriff mit dem Rastelement 42 gedrückt.

Somit dient der Kipphebel 56 nur zur kurzfristigen Aufhebung der Rastfunktion.

Soll diese Rastfunktion dauerhaft aufgehoben werden, wird das Abstellorgan 62 etwa um 90° seitlich verschwenkt, wie das aus dem Übergang von Fig. 4 zur Fig. 7 ersichtlich ist, und in Fig. 1 durch einen Pfeil 72 angedeutet ist.

Die Nockenfläche 68 ist so ausgebildet, dass diese in radialer Richtung ansteigt.

Wurde der Ring 64 in die in Fig. 7 dargestellte Position bewegt, wurde, wie das insbesondere in Fig. 6 ersichtlich ist, der zweite Hebelarm 52 soweit angehoben, dass der gegenüberliegende erste Hebelarm 46 in die Aussparung 39 hineinbewegt wird, also aus dem arretierenden Eingriff mit dem Rastelement 42 herausbewegt worden ist.

Diese Situation ist in Fig. 5 dargestellt.

Aus Fig. 5 ist auch ersichtlich, dass der in die Aussparung 39 hineinbewegte erste Hebelarm 46 den Kipphebel 56 bewegt hat, so dass sich die Taste 60 ebenfalls bewegt hat.

D.h., das Abstellorgan 62 übersteuert diesen Mechanismus, der durch den Kipphebel 56 bzw. die Taste 60 ausgelöst werden kann.

Diese Drehbewegung ist sehr ergonomisch durchzuführen, wie das aus dem Übergang von Fig. 4 zu Fig. 7 leicht verständlich wird.

In der zweiten Stellung steht der Stab 60, wie das insbesondere aus Fig. 7 ersichtlich ist, seitlich von dem beweglichen Griffteil 20 ab, schmiegt sich aber dennoch an die Unterseite des Gehäuses 16 an. Mit anderen Worten gesagt, in dieser Stellung sperrt der Stab 60 keine weitere Handhabung des Instrumentes 10.

Die in Fig. 5, 6 und 7 dargestellte Position entspricht der zweiten Stellung, d.h., in der die Rastfunktion auf Dauer aufgehoben ist.

Somit wirkt das Abstellorgan 62 als eine dauerhafte Rastsperre. Die durch den Pfeil 72 in Fig. 4 vorgegebene Ebene entspricht also der Drehebene 72 des Ringes, die quer vor dem Fingerring 41 des beweglichen Griffteiles 20 liegt.

Hat eine Handhabungsperson, wie zuvor beschrieben, das Instrument 10 mit einer Hand, beispielsweise mit der rechten Hand ergriffen, und hat den Mittelfinger in den Fingerring 41 eingeschoben, so kann mit dem Zeigefinger der Ring 46 durch Ergreifen des Stabes 66 mit dem Finger problemlos um 90° verdreht werden, also den Übergang von Fig. 4 zu Fig. 7 bewerkstelligen. Dabei muss das Instrument nicht freigegeben werden, und es steht beispielweise auch nach wie vor der Daumen zur Verfügung, um beispielsweise das Stellorgan 34 zu betätigen.

In dieser zweiten Stellung der Rastsperre kann nunmehr das bewegliche Griffteil 20 frei längs der Führung 32 hin- und herbewegt werden, wie das insbesondere aus der Schnittdarstellung von Fig. 9 ersichtlich ist.

Die Baueinheit, wie sie in Fig. 2 oder 5 dargestellt ist, also das bewegliche Griffteil 20 wird in der Fachsprache auch oft als Trigger bezeichnet.

In dieser zweiten Stellung, in der die Rastfunktion dauerhaft aufgehoben ist, kann beispielsweise der Operateur einen Präpariervorgang durchführen, indem er beispielsweise mit den Maulteilen 26 und 28 ein Gewebe oder eine Zyste oder ein Tumor entsprechend präpariert, sprich abtrennt. Nach Abtrennen des Gewebestückes soll dies zwischen den Maulteilen 26 und 28 gehalten werden, wozu dieses in eine ganz bestimmten Stellung gebracht werden soll, die dann unverändert sein soll, um sicherzustellen, dass das abgetrennte Gewebestück zwischen den beiden Maulteilen 26 und 28 festgehalten wird. Dazu wird dann die Rastsperre wieder aus der zweiten Stellung in die erste Stellung bewegt, also den Übergang von Fig. 7 nach Fig. 4.

Nunmehr kann der erste Hebelarm 46 mit seinen Rasthänden 48 wieder mit dem Rastelement einrasten und ein Hin- und Herbewegen des Triggers längs der Führung 32 ist gesperrt.

Nun können andere Handhabungen durchgeführt werden, beispielsweise falls der Schaft 12 seitlich auslenkt war, diesen zunächst mit dem Stellorgan 34 wieder in eine lineare ausgerichtete Position zu bringen, um anschließend den Schaft, der bei einem minimalinvasiven Eingriff durch einen Trokar in einer Körperhöhle eingeführt ist, wieder abzuziehen. Die Raste bleibt dabei permanent in ihrer verrasteten Stellung und stellt also sicher, dass die Maulteile 26 und 28 das dazwischen gehaltene Gewebe während dieser Manipulationen festhält und nicht verliert. Sollte die Handhabungsperson wünschen, beispielsweise den Anpressdruck zwischen den Maulteilen 26 und 28 etwas zu erhöhen, kann er den Trigger um einen Zahn weiterbewegen, um das bewegliche Griffteil 20 eine Zahnbreite zu bewegen.

Er kann selbstverständlich auch den umgekehrten Bewegungsvorgang durchführen, wenn er beispielweise feststellt, dass die Maulteile 26 und 28 das Gewebe zu fest halten, und die Gefahr besteht, dass das Gewebe in zwei Stücke aufgeteilt wird. Dann kann er durch kurzfristiges Drücken auf die Taste 60 sozusagen in die andere Richtung reagieren, eine Zahnbreite in die entgegengesetzte Richtung verschieben, um den Anpressdruck etwas zu verringern.

## Patentansprüche

1. Medizinisches Instrument, mit einem Schaft (12), der proximal mit einer Handhabe (14) verbunden ist, mit einem längs des Schaftes (12) geführten Einsatz (22), der proximalseitig mit einem beweglichen Griffteil (20) der Handhabe (14) verbunden ist und distal ein Werkzeug (24) aufweist, das durch Bewegen des Griffteiles (20) betätigbar ist, und mit einer Raste (40), durch die das bewegliche Griffteil (20) in einer Stellung arretierbar ist, wobei eine Rastsperre vorgesehen ist, durch die die Arretierfunktion dauerhaft aufhebbar ist, wobei die Rastsperre ein drehbares Abstellorgan (62) aufweist, das am beweglichen Griffteil (20) angeordnet ist, wobei das Abstellorgan (62) in einer ersten Drehstellung die Arretierfunktion der Raste (40) nicht beeinflusst, in einer zweiten Drehstellung die Raste (40) dauerhaft aus dem arretierenden Eingriff weghält, **dadurch gekennzeichnet, dass** die Drehebene (72) des Abstellorganes (62) quer zur Längsachse (74) der Bewegungsrichtung des Griffteiles (20) verläuft.

2. Medizinisches Instrument nach Anspruch 1, **dadurch gekennzeichnet, dass** das drehbare Abstellorgan (62) als Drehring (64) ausgebildet ist, der distal vor einem Fingerring (41) des beweglichen Griffteiles (20) angeordnet ist.

3. Medizinisches Instrument nach Anspruch 2, **dadurch gekennzeichnet, dass** vom Drehring (64) radial ein Stab (66) vorsteht, über den der Drehring (64) drehbar ist.

4. Medizinisches Instrument nach Anspruch 3, **dadurch gekennzeichnet, dass** sich der Stab (66) in der ersten Drehstellung in einer Ebene des Fingerringes (41) des beweglichen Griffteiles (20) erstreckt.

5. Medizinisches Instrument nach Anspruch 3 oder 4, **dadurch gekennzeichnet, dass** sich der Stab (66) in der zweiten Stellung seitlich vom beweglichen Griffteil (20) weg erstreckt.

6. Medizinisches Instrument nach einem der Ansprüche 1 bis 5, **dadurch gekennzeichnet, dass** ein Verschwenkbereich des drehbaren Abstellorganes ca. 90° beträgt.

7. Medizinisches Instrument nach einem der Ansprüche 1 bis 6, **dadurch gekennzeichnet, dass** im bewegbaren Griffteil (20) ein Mechanismus angeordnet ist, der in der ersten Drehstellung des Abstellorganes (62) eine Aufhebung der Arretierfunktion der Raste (40) ermöglicht.

8. Medizinisches Instrument nach Anspruch 7, **dadurch gekennzeichnet, dass** der Mechanismus einen Kipphebel (56) aufweist, der mit der Raste (40) in Wirkverbindung steht.

9. Medizinisches Instrument nach Anspruch 2 und 8, **dadurch gekennzeichnet, dass** der Kipphebel (56) eine im Fingerring (41) des beweglichen Griffteiles (20) angeordnete Taste (60) aufweist.

10. Medizinisches Instrument nach Anspruch 9, **dadurch gekennzeichnet, dass** ein Drücken der Taste (60) die Raste (40) aus der arretierenden Position abhebt und die Raste (40) bei Freigabe der Taste (60) wieder in die arretierende Position bewegt wird.

11. Medizinisches Instrument nach einem der Ansprüche 1 bis 10, **dadurch gekennzeichnet, dass** die Raste (40) als zweiarmiger Hebel (44) ausgebildet ist, wobei ein erster Hebelarm (46) Rastzähne (48) trägt, die mit einem Rastelement (42) an der Handhabe (14) in arretierenden Eingriff treten.

12. Medizinisches Instrument nach Anspruch 11, **dadurch gekennzeichnet, dass** das bewegbare Griffteil (20) längs einer linearen Führung (32) längs der Handhabe (14) bewegbar ist, und dass die Rastzähne (48) des zweiarmigen Hebels (44) dem Rastelement (42) zugewandt sind.

13. Medizinisches Instrument nach einem der Ansprüche 1 bis 12, **dadurch gekennzeichnet, dass** die Raste (40) durch eine Feder (54) derart vorgespannt ist, dass die Raste (40) in den arretierenden Eingriff gedrückt wird.

14. Medizinisches Instrument nach einem der Ansprüche 11 bis 13, **dadurch gekennzeichnet, dass** das drehbare Abstellorgan (62) eine umfängliche radial ansteigende Nockenfläche (68) aufweist, die beim Drehen aus der ersten in die zweite Drehstellung den ersten Hebelarm (46) mit den Rastzähnen (48) vom Rastelement (42) abhebt.

15. Medizinisches Instrument nach Anspruch 14, **dadurch gekennzeichnet, dass** die Nockenfläche (68) mit dem zweiten Hebelarm (52) des zweiarmigen Hebels (44) in Eingriff tretbar ist.

16. Medizinisches Instrument nach einem der Ansprüche 8 bis 15, **dadurch gekennzeichnet, dass** der Kipphebel (56) mit dem ersten Hebelarm (46) des zweiarmigen Hebels (44) in Eingriff steht.

## Claims

1. Medical instrument with a shaft (12) connected proximally to a handle (14), and with an insert (22) guided along the shaft (12), which insert is connected to a moveable grip part (20) of the handle (14) on the proximal side and distally has a tool (24) which can be actuated by moving the grip part (20), and with a lock (40) which can lock the moveable grip part (20) in a position, wherein a detent which can permanently lift the locking function is provided, the detent having a rotatable stopping member (62) arranged on the moveable grip part (20), with the stopping member (62) not influencing the locking function of the lock (40) in a first rotational position and permanently keeping the lock (40) away from a locking engagement in a second rotational position, **characterized in that** the rotational plane (72) of the stopping member (62) runs across the longitudinal axis (74) of the movement direction of the grip part (20).

2. Medical instrument of Claim 1, **characterized in that** the rotatable stopping member (62) is designed as a rotary ring (64) arranged distally in front of a finger ring (41) of the moveable grip part (20).

3. Medical instrument of Claim 2, **characterized in that** a rod (66) protrudes radially from the rotary ring (64) and it can be used to rotate the rotary ring (64).

4. Medical instrument of Claim 3, **characterized in that**, in the first rotational position, the rod (66) extends in a plane of the finger ring (41) of the moveable grip part (20).

5. Medical instrument of Claim 3 or 4, **characterized in that**, in the second position, the rod (66) extends laterally away from the moveable grip part (20).

6. Medical instrument of anyone of Claims 1 to 5, **characterized in that** the range through which the rotatable stopping member can pivot is approximately 90°.

7. Medical instrument of anyone of Claims 1 to 6, **characterized in that** a mechanism is arranged in the moveable grip part (20) and it allows the locking function of the lock (40) to be lifted in the first rotational position of the stopping member (62).

8. Medical instrument of Claim 7, **characterized in that** the mechanism has a rocker lever (56) which is operatively connected to the lock (40).

9. Medical instrument of Claims 2 and 8, **characterized in that** the rocker lever (56) has a pushbutton (60) arranged in the finger ring (41) of the moveable grip part (20).

10. Medical instrument of Claim 9, **characterized in that** pushing the pushbutton (60) lifts the lock (40) from the locking position and releasing the pushbutton (60) returns the lock (40) into the locking position.

11. Medical instrument of anyone of Claims 1 to 10, **characterized in that** the lock (40) is designed as a two-armed lever (44), wherein a first lever arm (46) supports latching teeth (48) engaging in a locking manner with a latching element (42) on the handle (14).

12. Medical instrument of Claim 11, **characterized in that** the moveable grip part (20) can be moved along a linear guide (32) along the handle (14), and **in that** the latching teeth (48) of the two-armed lever (44) face the latching element (42).

13. Medical instrument of anyone of Claims 1 to 12, **characterized in that** the lock (40) is pretensioned by a spring (54) such that the lock (40) is pushed into the locking engagement.

14. Medical instrument of anyone of Claims 11 to 13, **characterized in that** the rotatable stopping member (62) has a circumferential radially increasing cam face (68) which lifts the first lever arm (46) with the latching teeth (48) from the latching element (42) when rotated from the first rotational position into the second rotational position.

15. Medical instrument of Claim 14, **characterized in that** the cam face (68) can engage with the second lever arm (52) of the two-armed lever (44).

16. Medical instrument of anyone of Claims 8 to 15, **characterized in that** the rocker lever (56) engages with the first lever arm (46) of the two-armed lever (44).

## Revendications

1. Instrument médical comprenant un fût (12) relié à un manche (14) dans la région proximale ; une pièce intégrée (22) qui est guidée le long dudit fût (12), est reliée à une partie mobile de préhension (20) dudit manche (14) côté proximal, et est munie, dans la région distale, d'un outil (24) pouvant être actionné par mouvement imprimé à ladite partie de préhension (20) ; et un cran d'arrêt (40) par lequel ladite partie mobile de préhension (20) peut être bloquée à demeure dans une position, sachant qu'il est prévu un verrou à déclic par l'intermédiaire duquel la fonction de blocage à demeure peut être neutralisée durablement, ledit verrou à déclic présentant un organe rotatif de débrayage (62) disposé sur ladite partie mobile de préhension (20), lequel organe de débrayage (62) n'influence pas la fonction de blocage à demeure exercée par le cran d'arrêt (40), dans une première position prise par rotation, et interdit durablement, dans une seconde position prise par rotation, l'engagement dudit cran d'arrêt (40) avec effet de blocage à demeure, **caractérisé par le fait que** le plan de rotation (72) de l'organe de débrayage (62) s'étend transversalement par rapport à l'axe longitudinal (74) de la direction de mouvement de la partie de préhension (20).

2. Instrument médical selon la revendication 1, **caractérisé par le fait que** l'organe rotatif de débrayage (62) est réalisé sous la forme d'une bague tournante (64) située, dans la région distale, devant un pontet (41) de la partie mobile de préhension (20).

3. Instrument médical selon la revendication 2, **caractérisé par le fait qu'**une barrette (66), radialement en saillie au-delà de la bague tournante (64), permet à ladite bague tournante (64) d'accomplir une rotation.

4. Instrument médical selon la revendication 3, **caractérisé par le fait que** la barrette (66) s'étend, dans la première position prise par rotation, dans un plan du pontet (41) de la partie mobile de préhension (20).

5. Instrument médical selon la revendication 3 ou 4, **caractérisé par le fait que** la barrette (66) s'étend, dans la seconde position, latéralement à distance de la partie mobile de préhension (20).

6. Instrument médical selon l'une des revendications 1 à 5, **caractérisé par le fait qu'**une plage de pivotements de l'organe rotatif de débrayage mesure environ 90°.

7. Instrument médical selon l'une des revendications 1 à 6, **caractérisé par le fait que** la partie mobile de préhension (20) renferme un mécanisme autorisant, dans la première position que l'organe de débrayage (62) a prise par rotation, une neutralisation de la fonction de blocage à demeure exercée par le cran d'arrêt (40).

8. Instrument médical selon la revendication 7, **caractérisé par le fait que** le mécanisme comporte un levier basculant (56) en liaison opérante avec le cran d'arrêt (40).

9. Instrument médical selon les revendications 2 et 8, **caractérisé par le fait que** le levier basculant (56) présente un poussoir (60) logé dans le pontet (41) de la partie mobile de préhension (20).

10. Instrument médical selon la revendication 9, **caractérisé par le fait qu'**un enfoncement du poussoir (60) soulève le cran d'arrêt (40) à l'écart de l'emplacement de blocage à demeure, et ledit cran d'arrêt (40) est ramené audit emplacement de blocage à demeure lors d'un relâchement dudit poussoir (60).

11. Instrument médical selon l'une des revendications 1 à 10, **caractérisé par le fait que** le cran d'arrêt (40) est réalisé sous la forme d'un levier (44) à deux bras, un premier bras (46) dudit levier portant des dents de crantage (48) qui viennent en prise, en exerçant un effet de blocage à demeure, avec un élément de crantage (42) situé sur le manche (14).

12. Instrument médical selon la revendication 11, **caractérisé par le fait que** la partie mobile de préhension (20) peut être mue le long du manche (14), en longeant un guide rectiligne (32) ; et **par le fait que** les dents de crantage (48) du levier (44) à deux bras pointent vers l'élément de crantage (42).

13. Instrument médical selon l'une des revendications 1 à 12, **caractérisé par le fait que** le cran d'arrêt (40) est précontraint par un ressort (54), de telle sorte que ledit cran d'arrêt (40) soit poussé jusqu'à la venue en prise exerçant un effet de blocage à demeure.

14. Instrument médical selon l'une des revendications 11 à 13, **caractérisé par le fait que** l'organe rotatif de débrayage (62) comporte une surface de came (68) à pente radialement ascendante, qui est située sur le pourtour et qui, lors de la rotation s'opérant de la première à la seconde position prise par rotation, soulève le premier bras de levier (46) à l'écart de l'élément de crantage (42) conjointement aux dents de crantage (48).

15. Instrument médical selon la revendication 14, **caractérisé par le fait que** la surface de came (68) peut être mise en prise avec le second bras (52) du levier (44) à deux bras.

16. Instrument médical selon l'une des revendications 8 à 15, **caractérisé par le fait que** le levier basculant (56) est en prise avec le premier bras (46) du levier (44) à deux bras.
